# EUROPEAN PATENT SPECIFICATION

(11) **EP 3 151 808 B1**
(45) Date of publication and mention of the grant of the patent: **17.10.2018**
(21) Application number: 15724699.2
(22) Date of filing: 28.05.2015
(51) Int. Cl.: A61K 8/24, A61Q 11/00, A61K 8/19, A61K 8/25

(54) **ORAL CARE COMPOSITIONS**
MUNDPFLEGEZUSAMMENSETZUNGEN
COMPOSITIONS POUR SOINS BUCCAUX

(30) Priority: 09.06.2014 WO PCT/CN2014/079466
(43) Date of publication of application: 12.04.2017
(73) Proprietor: Unilever PLC, London Greater London EC4Y 0DY (GB); Unilever NV, 3013 AL Rotterdam (NL)
(72) Inventor: LE, Ying, Shanghai 200335 (CN); LI, Xiaoke, Shanghai 200335 (CN); ZENG, Xiaoyu, Shanghai 200335 (CN)
(74) Representative: Tansley, Sally Elizabeth
(86) International application number: PCT/EP2015/061814
(87) International publication number: WO 2015/189041

(56) References cited:
- EP-B1- 2 089 040
- WO-A1-2012/143220
- US-A- 5 895 641

## Description

### FIELD OF THE INVENTION

The present invention relates to tooth-remineralizing oral care composition as well as a method for remineralizing teeth. More particularly the present invention relates to such compositions and methods which employ high levels of re-mineralizing agents.

### BACKGROUND OF THE INVENTION

Acidic foods and drinks can lead to tooth erosion by attacking enamel that coats and protects the teeth. In addition to what we consume, the natural equilibrium between tooth hydroxyapatite being dissolved from the enamel of teeth and hydroxyapatite being formed on or in teeth from substances occurring naturally in the saliva shifts continuously.

Products that address enamel erosion have, nevertheless, been developed.

EP 2 089 040 describes oral care compositions which remineralises eroded teeth and/or whitens the teeth. The composition can be a dual phase composition comprising a source of calcium ions, a source of phosphate ions. With such systems it is preferable if the level of water within the composition is low.

There remains a need for an oral care product that is stable and comprises a high level of materials that can remineralise tooth enamel.

### SUMMARY OF THE INVENTION

The present invention relates to an oral care product according to claim 1.

The invention also relates to a cosmetic method of treating teeth in which the first and second composition described above are mixed prior to their application to the teeth.

The invention further relates to a cosmetic product as described above for use in providing at least one benefit to the teeth of an individual, said benefit selected from remineralization of enamel, anti-erosion of enamel, decreased sensitivity, anticaries and combinations thereof.

In the context of the present invention an aqueous based composition means a composition comprising greater than 20 wt% of the total composition of water.

### DETAILED DESCRIPTION OF THE INVENTION

Types of calcium salt ("remineralising calcium sources) suitable for use with the present invention is calcium silicate.

The amount of silicate in the first composition preferably ranges from 25 to 80 wt% of the total first composition more preferably from 28 to 50 wt%.

Preferably the first composition comprises less 0.1 percent by weight of phosphate ions:
Preferably the first composition has a pH of from 8.5 to 11.

Preferably the calcium silicate has a Ca: Si ratio of from 1:5 to 3:1.

The second composition according to the invention comprises potassium phosphate salt.

Preferred potassium phosphate salts include, potassium dihydrogenphosphate, tripotassium phosphate, dipotassium hydrogen phosphate and mixtures thereof. Preferably the potassium phosphate comprises potassium dihydrogen phosphate, dipotassium hydrogen phosphate or mixtures thereof, mixtures being more preferred. It is advantageous if the weight ratio of dipotassium hydrogen phosphate to potassium dihydrogen phosphate is greater than 2:1, more preferably the weight ratio of dipotassium hydrogen phosphate to potassium dihydrogen phosphate is from 3:1 to 5:1.

In the context of the present invention the phosphate salts listed above may be hydrated. The total weight of potassium phosphate in the second composition is from 40 to 60 wt%.

Preferably the weight of water in the second composition is from 30 to 60 wt% of the total second composition.

Other phosphate sources may be used in the second composition, but this is not a preferable option. Such sources include, for example, sodium dihydrogen phosphate, disodium hydrogen phosphate, sodium pyrophosphate, tetrasodium pyrophosphate, sodium tripolyphosphate, sodium hexametaphosphate, trisodium phosphate, and mixtures thereof.

Compositions according to the invention may contain further ingredients to enhance performance and/or consumer acceptability such as abrasive cleaning agent and surfactant.

The compositions may comprise an abrasive cleaning agent in an amount of from 3 to 75% by weight based on the total weight of the dentifrice. Suitable abrasive cleaning agents include particulate abrasive materials such as abrasive silicas, aluminas, calcium carbonates, zirconium silicate, polymethylmethacrylate, dicalcium phosphates, calcium pyrophosphates, hydroxyapatites, trimetaphosphates, insoluble hexametaphosphates and agglomerates and/or mixtures thereof.

The composition may comprise a surfactant in an amount of from 0.2 to 5% by weight based on the total weight of the dentifrice. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

The composition may comprise a fluoride source such as sodium fluoride, stannous fluoride, sodium monofluorophosphate, zinc ammonium fluoride, tin ammonium fluoride, calcium fluoride, cobalt ammonium fluoride and mixtures thereof.

Other ingredients that may be present include:
plant-derivable antioxidants such as flavonoid, catechin, polyphenol, and tannin compounds and mixtures thereof; antioxidant vitamins such as tocopherols and/or derivatives thereof, ascorbic acid and/or derivatives thereof and mixtures thereof.

Compositions of the invention may be in any format and are preferably gels or liquids.

Compositions of the invention may be thickened, preferred thickening agents include polysacccaarides such as cellulose gum and thickening silicas.

In use it is preferable to combine the first and second composition to form a mix prior to their application to the teeth. Preferably the weight of the first composition to the second composition in this mix is greater than 2:1, more preferably from 2.5:1 to 5:1.

Preferably the level of calcium silicate in the mix is greater than 17 wt% of the composition more preferably greater than 20 wt% of the total mix composition.

Preferably the level of phosphate in the mix is greater than 6 wt% of the total mix composition, more preferably greater than 8 wt% of the total mix composition.

It is highly preferable if the calcium salt and potassium phosphate salt are precursors for *in-situ* hydroxyapatite formation.

The invention will now be illustrated by the following non-limiting Examples. Examples according to the invention are illustrated by a number, comparative Examples are illustrated by a letter.

### Examples

Compositions were made according to Table 1.

**Table 1**

| **COMPOSITION 1** | |
|---|---|
| **INGREDIENT** | **% w/w** |
| Water | 21.4000 |
| Sorbitol70%(non crystallising) | 45.0000 |
| PEG 400 | 2.0000 |
| Calcium Silicate | 30.0000 |
| Thickening Silica(medium vis.) | 0.5000 |
| SCMC 9H-3000/Cellulose Gum | 0.3000 |
| Optamint 405213 | 0.3000 |
| Benzyl Alcohol | 0.5000 |
| TOTAL | 100.00 |
| | |

| **COMPOSITION 2** | |
|---|---|
| **INGREDIENT** | **% w/w** |
| Water | 48.100 |
| Dipotassium Hydrogen Phosphate Trihydrate | 39.5000 |
| Potassium Dihydrogen Phosphate | 11.900 |
| Benzyl Alcohol | 0.5000 |
| TOTAL | 100.00 |

Compositions were mixed to give the differing weights of potassium salts and silicates in a single composition:
Compositions were assessed for enamel remineralisation as follows:
5mm by 5mm enamel blocks were cut from sound bovine incisors. The blocks were then embedded in dental base acrylic resin. The enamel surface of the resin blocks were then exposed with 800 grit paper and then polished flat using 1200, 1500 and then 2000 grit paper and finally 3um diamond polish. The blocks were then rinsed under di water and allowed to air dry before an initial indentation, primarily to ensure that the enamel has sufficient mineral hardness for the study (minimum acceptance criterion of SMH>300). The enamel was then stored at 4°C in di water until required.

36 enamel were selected to give n=6 enamel per treatment. Every 6 enamels were placed into a measuring cup and covered with 20ml 1%w/w citric acid solution at pH2.20 for ten minutes. Then the enamels were removed and rinsed in copious amounts of fresh water and left to soak in fresh water at 4 °C before the erosive lesion microhardness measurements were made.

At each indentation measurement point, five separate indents were made per sample and the average value recorded. Indentation was made using a certified Knoop diamond under a load of 50g.

Compositions phase 1 and phase 2 were mixed thoroughly to give the required ratio of calcium silicate to phosphate salt; for control group gels were made according to the table above. The total weight of gel is 4g in each group. The gels were applied onto enamel within five minutes for five minutes before being rinsed off with di water. Then, the blocks were incubated in SOF at 37°C for 24 hours. The microhardness was measured at instant, 4 hours and 24 hours after treatment.

**Table 2**

| **Ex.** | **Active Level** | | **Sound Enamel (SMH)** | **after acid (SMH)** | **After 5 mins product treatment** | | **4h after treatment** | | **24h after treatment** | |
|---|---|---|---|---|---|---|---|---|---|---|
| | **CaSi** | **P** | | | **SMH** | **p value*** | **SMH** | **p value*** | **SMH** | **p value*** |
| **1** | 15 | 7 | 338.17 | 133.57 | 140.30 | 0.9815 | 147.60 | 0.7855 | 158.70 | 0.2784 |
| **2** | 15 | 10.5 | 348.73 | 139.07 | 157.17 | 0.7709 | 153.30 | 0.8881 | 168.30 | 0.3530 |
| **3** | 22.5 | 7 | 347.90 | 123.63 | 139.00 | 0.8282 | 135.37 | 0.9268 | 153.13 | 0.2853 |
| **4** | 22.5 | 10.5 | 344.73 | 120.81 | 146.97 | 0.0584 | 149.72 | 0.0301 | 170.67 | 0.0001 |
| **A** | 0 | 0 | 344.17 | 132.90 | 122.18 | 0.8948 | 110.46 | 0.3533 | 115.93 | 0.6009 |

Active level is the total level of CaSi or phosphate salt in the final mix of the formulation.

Compositions showed good stability on storage.

Example 4 gave the best 4 and 24 hour enamel regeneration.

An attempt was made to replicate this data with salts of sodium hydrogen phosphate, however formulation were too difficult to manufacture.

**Table 3**

| **Formulation 1** | | **Formulation 2** | |
|---|---|---|---|
| **Material** | **w/w%** | **Material** | **w/w** |
| Calcium silicate | 35.9 | Potassium phosphate | 45.4 |
| Sodiumcarboxymethyl cellulose | 0.4 | Alginate | 4.3 |
| Sorbitol | 34.8 | Calcium hydrogen phosphate | 8.7 |
| Sodium lauryl sulphate | 11.5 | Sodium lauryl sulphate | 4.3 |
| Water | To 100 | Water | To 100 |

Formulation 1 and 2 were mixed in equal amounts and used to brush extracted human teeth two times a day for 4 weeks.

Teeth were cross-sectioned after the procedure, and then scanned using scanning electronic microscopy. After the procedure a new layer of material was seen on the surface of the teeth.

## Claims

1. An oral care product comprising a first composition comprising from 25 to 80 wt% of the total first composition of a calcium salt which is calcium silicate, and a second aqueous composition comprising greater than 20 wt% of the total second composition of water and from 40 to 60 wt% of the total second composition of a potassium phosphate salt.

2. An oral care product according to claim 1 in which the potassium phosphate salt comprises dipotassium hydrogen phosphate.

3. An oral care product according to claim 1 in which the potassium phosphate salt comprises potassium dihydrogen phosphate.

4. An oral care product according to claim 3 in which the potassium phosphate salt comprises a mixture of dipotassium hydrogen phosphate and potassium dihydrogen phosphate.

5. An oral care product according to claim 4 in which the weight ratio of dipotassium hydrogen phosphate to potassium dihydrogen phosphate is greater than 2:1.

6. An oral care product according to claim 5 in which the weight ratio of dipotassium hydrogen phosphate to potassium dihydrogen phosphate is from 3:1 to 5:1.

7. An oral care product according to any preceding claim in which the total weight of water in the second composition is from 30 to 60 wt% of the total composition.

8. An oral care product according to claim 1, wherein the calcium silicate has a Ca: Si ratio of from 1:5 to 3:1.

9. An oral care product according to any of the preceding claims, wherein the first composition comprises less 0.1 percent by weight of phosphate ions.

10. An oral care product according to any of the preceding claims, wherein the first composition has a pH of from 8.5 to 11.

11. An oral care product according to any preceding claim in which the calcium salt and potassium phosphate salt are precursors for *in-situ* hydroxyapatite formation.

12. An oral care product for use in the treatment of teeth, the product comprising a first and second composition according to any of the preceding claims which are combined to form a mix prior to their application to the teeth.

13. An oral care product for use according to claim 12 in which the level of calcium silicate in the mix is greater than 20 wt% of the total mix composition.

14. An oral care product for use according to claims 12 or 13 in which the level of phosphate in the mix is greater than 8 wt% of the total mix composition.

15. An oral care product according to any one of claims 1 to 12 for use in providing at least one benefit to the teeth of an individual, said benefit selected from remineralization of enamel, anti-erosion of enamel, decreased sensitivity, anticaries and combinations thereof.

## Patentansprüche

1. Mundpflegeprodukt, umfassend eine erste Zusammensetzung, umfassend von 25 bis 80 Gew.-% der gesamten ersten Zusammensetzung an einem Calciumsalz, das Calciumsilikat ist, und eine zweite wässrige Zusammensetzung, umfassend mehr als 20 Gew.-% der gesamten zweiten Zusammensetzung an Wasser und von 40 bis 60 Gew.-% der gesamten zweiten Zusammensetzung an einem Kaliumphosphatsalz.

2. Mundpflegeprodukt nach Anspruch 1, in welchem das Kaliumphosphatsalz Dikaliumhydrogenphosphat umfasst.

3. Mundpflegeprodukt nach Anspruch 1, in welchem das Kaliumphosphatsalz Kaliumdihydrogenphosphat umfasst.

4. Mundpflegeprodukt nach Anspruch 3, in welchem das Kaliumphosphatsalz eine Mischung von Dikaliumhydrogenphosphat und Kaliumdihydrogenphosphat umfasst.

5. Mundpflegeprodukt nach Anspruch 4, in welchem das Gewichtsverhältnis des Dikaliumhydrogenphosphats zum Kaliumdihydrogenphosphat größer als 2:1 ist.

6. Mundpflegeprodukt nach Anspruch 5, in welchem das Gewichtsverhältnis des Dikaliumhydrogenphosphats zum Kaliumdihydrogenphosphat von 3:1 bis 5:1 beträgt.

7. Mundpflegeprodukt nach irgendeinem vorhergehenden Anspruch, in welchem das Gesamtgewicht des Wassers in der zweiten Zusammensetzung von 30 bis 60 Gew.-% der gesamten Zusammensetzung beträgt.

8. Mundpflegeprodukt nach Anspruch 1, wobei das Calciumsilikat ein Ca:Si-Verhältnis von 1:5 bis 3:1 aufweist.

9. Mundpflegeprodukt nach irgendeinem der vorhergehenden Ansprüche, wobei die erste Zusammensetzung weniger als 0,1 Gewichtsprozent Phosphationen umfasst.

10. Mundpflegeprodukt nach irgendeinem der vorhergehenden Ansprüche, wobei die erste Zusammensetzung einen pH von 8,5 bis 11 aufweist.

11. Mundpflegeprodukt nach irgendeinem vorhergehenden Anspruch, in welchem das Calciumsalz und das Kaliumphosphatsalz Vorstufen einer in-situ-Hydroxyapatit-Bildung sind.

12. Mundpflegeprodukt zur Verwendung bei der Behandlung von Zähnen, wobei das Produkt eine erste und eine zweite Zusammensetzung nach irgendeinem der vorhergehenden Ansprüche umfasst, die kombiniert werden, um vor deren Auftragen auf die Zähne eine Mischung zu bilden.

13. Mundpflegeprodukt zur Verwendung nach Anspruch 12, in welchem der Gehalt des Calciumsilikats in der Mischung größer als 20 Gew.-% der gesamten Mischungszusammensetzung ist.

14. Mundpflegeprodukt zur Verwendung nach den Ansprüchen 12 oder 13, in welchen der Gehalt des Phosphats in der Mischung größer als 8 Gew.-% der gesamten Mischungszusammensetzung ist.

15. Mundpflegeprodukt nach irgendeinem der Ansprüche 1 bis 12 zur Verwendung in der Schaffung mindestens eines Nutzens für die Zähne eines Individuums, wobei der Nutzen unter der Remineralisierung von Zahnschmelz, der Antierosion von Zahnschmelz, verminderter Empfindlichkeit, Antikaries und Kombinationen davon ausgewählt ist.

## Revendications

1. Produit pour le soin oral comprenant une première composition comprenant de 25 à 80 % en masse de la première composition totale d'un sel de calcium qui est le silicate de calcium, et une seconde composition aqueuse comprenant plus de 20 % en masse de la seconde composition totale d'eau et de 40 à 60 % en masse de la seconde composition totale d'un sel de phosphate de potassium.

2. Produit pour le soin oral selon la revendication 1 dans lequel le sel de phosphate de potassium comprend de l'hydrogénophosphate de dipotassium.

3. Produit pour le soin oral selon la revendication 1 dans lequel le sel de phosphate de potassium comprend du dihydrogénophosphate de potassium.

4. Produit pour le soin oral selon la revendication 3 dans lequel le sel de phosphate de potassium comprend un mélange d'hydrogénophosphate de dipotassium et de dihydrogénophosphate de potassium.

5. Produit pour le soin oral selon la revendication 4 dans lequel le rapport massique d'hydrogénophosphate de dipotassium à dihydrogénophosphate de potassium est supérieur à 2:1.

6. Produit pour le soin oral selon la revendication 5, dans lequel le rapport massique d'hydrogénophosphate de dipotassium à dihydrogénophosphate de potassium est de 3:1 à 5:1.

7. Produit pour le soin oral selon l'une quelconque des revendications précédentes, dans lequel la masse totale d'eau dans la seconde composition est de 30 à 60 % en masse de la composition totale.

8. Produit pour le soin oral selon la revendication 1, dans lequel le silicate de calcium présente un rapport Ca:Si de 1:5 à 3:1.

9. Produit pour le soin oral selon l'une quelconque des revendications précédentes, dans lequel la première composition comprend moins de 0,1 % en masse d'ions phosphate.

10. Produit pour le soin oral selon l'une quelconque des revendications précédentes, dans lequel la première composition présente un pH de 8,5 à 11.

11. Produit pour le soin oral selon l'une quelconque des revendications précédentes, dans lequel le sel de calcium et le sel de phosphate de potassium sont des précurseurs pour une formation *in-situ* d'hydroxyapatite.

12. Produit pour le soin oral pour une utilisation dans le traitement des dents, le produit comprenant une première et seconde composition selon l'une quelconque des revendications précédentes qui sont combinées pour former un mélange avant leur application aux dents.

13. Produit pour le soin oral pour une utilisation selon la revendication 12, dans lequel la teneur en silicate de calcium dans le mélange est supérieure à 20 % en masse de la composition de mélange totale.

14. Produit pour le soin oral pour une utilisation selon les revendications 12 ou 13, dans lequel la teneur en phosphate dans le mélange est supérieure à 8 % en masse de la composition de mélange totale.

15. Produit pour le soin oral selon l'une quelconque des revendications 1 à 12 pour une utilisation fournissant au moins un bénéfice aux dents d'individu, ledit bénéfice choisi parmi une reminéralisation de l'émail, une anti-érosion de l'émail, une sensibilité abaissée, des anticaries et des combinaisons de ceux-ci.
